# EUROPEAN PATENT SPECIFICATION

(11) **EP 1 572 109 B1**
(45) Date of publication and mention of the grant of the patent: **27.06.2007**
(21) Application number: 03787226.4
(22) Date of filing: 26.11.2003
(51) Int. Cl.: A61K 6/00, A61K 6/08, C08F 4/32, C08F 4/40

(54) **FREE-RADICAL INITIATOR SYSTEMS CONTAINING ENZYMES**
RADIKALINITIATORSYSTEME ENTHALTEND ENZYME
SYSTEMES INITIATEURS A RADICAUX LIBRES CONTENANT DES ENZYMES

(30) Priority: 20.12.2002 US 327202
(43) Date of publication of application: 14.09.2005
(73) Proprietor: 3M Innovative Properties Company, St. Paul, Minnesota 55133-3427 (US)
(72) Inventor: WU, Dong,, Saint Paul, MN 55133-3427 (US); OXMAN, Joel D.,, Saint Paul, MN 55133-3427 (US)
(74) Representative: Vossius & Partner
(86) International application number: PCT/US2003/038147
(87) International publication number: WO 2004/060324

(56) References cited:
- EP-A- 0 321 872
- WO-A-01/37787
- TAIRA Y ET AL: "A STUDY ON CYTOCHROME C OXIDOREDUCTASE FOR BONDING A TRI-N-BUTYLBORANE-INITIATED LUTING AGENT TO DENTIN" JOURNAL OF BIOMEDICAL MATERIALS RESEARCH, WILEY, NEW YORK, NY, US, vol. 48, no. 5, 1999, pages 697-699, XP000997686 ISSN: 0021-9304

## Description

Various polymerizable compositions include free-radical initiator systems for redox polymerization. Such compositions are commonly used in medical and dental applications. However, many include the use of oxidizing agents, such as peroxides and persulfates, which can be unstable and cause problems with color stability of the compositions. Certain additives for such compositions can improve color stability; however, some useful additives that improve color stability can increase the potential toxic and/or narcotic properties of these compositions. Thus, there is a need for compositions that are more medically acceptable.

EP-A-0 321 872 discloses a method of polymerizing or copolymerizing of free-radically polymerizable monomers dissolved or dispersed in aqueous systems using a free-radical initiator which is in-situ generated in the polymerization system by adding a peroxide generating enzyme and one or more substrates for the enzyme in the presence of oxygen.

The present invention provides a free-radical initiator system that includes a combination of an oxidase enzyme, a peroxidase coenzyme, an oxidase substrate, and a reductant (i.e., reducing agent). This system preferably provides relatively rapid free-radical initiation of a resin system that includes a polymerizable component without the use of unstable oxidizing agents. Furthermore, such systems are more suitable for medical and dental applications than many conventional systems because of their relatively low toxicity.

In a preferred embodiment, the present invention provides a free-radical initiator system that includes: an oxidase and a peroxidase; an oxidase substrate; and a reducing agent selected from the group consisting of sulfinic acid salts, ascorbic acid, amino acids (preferably, amino acids selected from the group consisting of cysteine, N-phenylglycine, histadine, and combinations thereof), barbituric acid derivatives, and combinations thereof.

In another preferred embodiment, the present invention provides a free-radical initiator system that includes: an oxidase and a peroxidase; an oxidase substrate; and a reducing agent that at least partially hardens a mixture of polyethyleneglycol dimethacrylate having a molecular weight of about 400, water, glucose, glucose oxidase, and horseradish peroxidase in no greater than 60 minutes at about 37°C (tested per the Polymerization Test Method described in the Examples Section). Preferably, this reducing agent may be selected from sulfinic acid salts, ascorbic acid, amino acids, barbituric acid derivatives, and combinations thereof.

The present invention also provides a composition (preferably, a dental composition) that includes a resin system, which includes a polymerizable component, and a free-radical initiator system as described herein. Preferably, the polymerizable component includes an ethylenically unsaturated component. Particularly for dental compositions, the polymerizable component includes a multifunctional component. More preferably, the resin system may be selected from ethylenically unsaturated compounds such as (meth)acrylates, (meth)acrylamides, and combinations thereof.

In certain embodiments, the composition can optionally include water and optionally a cosolvent.

The compositions of the present invention, particularly the dental compositions, can optionally be in the form of two or more parts, both of which, for example, can be in the form of pastes.

A particularly preferred dental composition of the present invention includes: a resin system including a polymerizable component (preferably, an ethylenically unsaturated polymerizable component, and more preferably a multifunctional ethylenically unsaturated polymerizable component); and a free-radical initiator system that includes: an oxidase and a peroxidase; an oxidase substrate; and a reducing agent that at least partially hardens a mixture of polyethyleneglycol dimethacrylate having a molecular weight of about 400, water, glucose, glucose oxidase, and horseradish peroxidase in no greater than 60 minutes at about 37°C (tested per the Polymerization Test Method described in the Examples Section). Preferably, the reducing agent may be selected from sulfinic acid salts, ascorbic acid, amino acids, barbituric acid derivatives, and combinations thereof.

The present invention also provides methods for preparing hardened compositions. The methods include combining a resin system, which includes a polymerizable component, and a free-radical initiator system under conditions effective to harden the composition, wherein the free-radical initiator system is described herein. Preferably, the conditions effective to harden the composition may include a temperature of 0°C to 60°C. Even more preferably, the components of the composition are selected such that the composition hardens at a temperature of about 25°C in less than 120 minutes.

The present invention provides a free-radical initiator system for use with a resin system in various hardenable compositions. The free-radical initiator system includes a combination of an oxidase enzyme, a peroxidase coenzyme, an oxidase substrate, and a reductant (i.e., reducing agent). This system preferably provides relatively rapid free-radical initiation without the use of unstable oxidizing agents.

The compositions of the present invention include the free-radical initiator system, the resin system, and optional components such as water, a cosolvent, a filler, a photoinitiator, a surfactant, as well as other optional additives well known to those of skill in the art.

The free-radical initiator systems, and hence, the hardenable compositions, of the present invention are particularly suitable for medical and dental materials, particularly dental materials. Medical materials include, for example, tissue sealants, scaffolding materials, and treatments for ulcerated tissues. Dental materials include, for example, sealants, restoratives, hard and/or soft tissue coatings, prosthodontic devices, cements, adhesives, and periodontal treatments.

The free-radical initiator system includes: an oxidase and a peroxidase; an oxidase substrate; and a reducing agent. The oxidase, peroxidase, and reducing agent should cooperate with one another to produce free radicals capable of initiating polymerization of the resin system. This type of cure is a dark reaction. That is, it is not dependent on the presence of light and can proceed in the absence of light.

The enzymes and reducing agent are preferably sufficiently shelf-stable to permit their storage and use under typical dental conditions. They should be sufficiently miscible with the resin system (and preferably water-soluble) to permit ready dissolution in (and discourage separation from) the other components of the hardenable composition.

The enzymes suitable for use in the present invention are oxidoreductase enzymes. These are enzymes classified under the Enzyme Classification number E.C. 1 in accordance with the Recommendations (1992) of the International Union of Biochemistry and Molecular Biology (IUBMB). These enzymes catalyze oxidoreductions (i.e., redox reactions). Within the group of oxidoreductase enzymes there are oxidase and peroxidase enzymes.

Oxidase enzymes catalyze the oxidation of a substrate by acting on O₂ as an acceptor of electrons and forming hydrogen peroxide. Such enzymes are classified under the enzyme classification E.C. 1.1.3, E.C. 1.2.3, E.C. 1.3.3, E.C. 1.4.3, E.C. 1.5.3. E.C. 1.7.3, E.C. 1.8.3, E.C. 1.9.3. Examples include, but are not limited to, glucose oxidase, sucrose oxidase, lactate oxidase, (S)-2-hydroxy-acid oxidase, hexose oxidase, L-or D-amino-acid oxidase, xylitol oxidase, xanthine oxidase, glycolate oxidase, L-sorbose oxidase, alcohol oxidase, gulonolactone oxidase. Various combinations of oxidase enzymes can be used according to the present invention. Preferably, the oxidase may be glucose oxidase, lactate oxidase, hexose oxidase, glycolate oxidase, gulonolactone oxidase, L-sorbose oxidase, (S)-2-hydroxy-acid oxidase, xanthine oxidase, or combinations thereof.

Peroxidase enzymes act on peroxide as an acceptor of electrons. Such enzymes are classified under enzyme classification E.C. 1.11. The different types of peroxidase enzymes are distinguished by the donor molecules from which they take electrons to donate to hydrogen peroxide. In accordance with the present invention a peroxidase is used to generate free radicals from the donor molecules. The donor molecules are typically capable of acting as a substrate for the peroxidase in generating such free radicals. Various combinations of peroxidase enzymes can be used according to the present invention: Examples include, but are not limited to, horseradish peroxidase, soybean peroxidase, polyphenol peroxidase, manganese peroxidase, L-ascorbate peroxidase, chloroperoxidase, and iodide peroxidase. Preferably, the peroxidase may be horseradish peroxidase, polyphenol peroxidase, manganese peroxidase, soybean peroxidase, chloroperoxidase, or combinations thereof.

The free-radical initiator system can also include various combinations of oxidase enzyme substrates according to the present invention. Corresponding enzyme substrates for oxidase enzymes (referred to herein as an oxidase substrate) include, but are not limited to, β-D-glucose, sucrose, lactate, (S)-2-hydroxy-acid, broad spectrum of carbohydrates including D-glucose, D-galactose, D-marinose, maltose, lactose, and cellobiose, etc., L-or D-amino acids, xylitol, xanthine, α-hydroxy acids, L-sorbose, a primary alcohol, and L-gulonb-1,4-lactone. Preferably, the oxidase substrate includes glucose, lactate, hexose, gulonolactone, L-sorbose, (S)-2-hydroxy acid, xanthine, or combinations thereof.

The reducing agent is selected such that the agent at least partially hardens a mixture of polyethyleneglycol dimethacrylate having a molecular weight of about 400, water, glucose, glucose oxidase, and horseradish peroxidase in no greater than 60 minutes at about 37°C (tested per the Polymerization Test Method described in the Examples Section). Suitable reducing agents of the present invention that meet this test may be selected from a wide variety of commonly used reducing agents as described below.

Reducing agents of the present invention may or may not be polymerizable. Combinations of two or more reducing agents may be used to provide an optimum balance of working and polymerization characteristics as well as the final properties of the hardened material. The reducing agents can be in the form of a monomer, oligomer, or polymer. Various combinations of reducing agents can be used according to the present invention.

Nonpolymerizable reducing agents may include ascorbic acid and derivatives thereof, amines, tertiary amines, amino acids, barbituric acid and derivatives thereof, salts of a dithionite or sulfite anion, sulfinic acids, and sulfinic acid salts. Other nonpolymerizable reducing agents may include a urea or thiourea functionality. Other nonpolymerizable reducing agents may include low valent metal salts, e.g., salts of copper (I), iron (II), and cobalt (II). Various mixtures of nonpolymerizable reducing agents can be used if desired.

Polymerizable reducing agents may include acrylated tertiary amines, e.g., 2-dimethylaminoethyl (meth)acrylate. Another class of polymerizable reducing agents may include a urea or thiourea group. Urea and thiourea groups are known to function as reductants in oxidation-reduction (i.e., redox) polymerization reactions. In addition, derivatives of urea and thiourea may be useful as polymerizable reducing agents as described in U.S. Publication No. 2003/0166740, published September 4, 2003. Various combinations of such polymerizable reducing agents can be used if desired.

For certain embodiments, preferably the reducing agent may be selected from sulfinic acid salts, ascorbic acid, amino acids, barbituric acid derivatives, or combinations thereof. Preferably, suitable sulfinic acid salts include aromatic sulfinic acid salts such as sodium benzenesulfinate and sodium toluenesulfinate. Preferably, suitable amino acids include N-phenylglycine, histidine, and cysteine. Preferably, the barbituric acid derivatives include 1,3-dimethyl barbituric acid.

In certain embodiments, the initiator system can include oxygen. That is, the compositions of the present invention can be hardened in air. It is believed that this is due to the capability of the free-radical initiator system to consume oxygen and prevent oxygen inhibition, which is a common problem associated with free-radical polymerization.

The enzymes, enzyme substrate, and reducing agent are present in amounts sufficient to permit an adequate free-radical reaction rate. This can be evaluated by combining the ingredients of the hardenable composition and observing whether or not a hardened mass is obtained, preferably, in no greater than 60 minutes at about 37°C (tested per the Polymerization Test Method described in the Examples Section).

Preferably, the oxidase enzyme is present in an amount of at least 5 units, more preferably at least 10 units, and even more preferably at least 20 units, of enzyme per gram of polymerizable compound used in the hardenable composition. Preferably, the oxidase enzyme is present in an amount of no greater than 2000 units per gram of polymerizable compound used in the hardenable composition.

Preferably, the peroxidase enzyme is present in an amount of at least 25 units, more preferably at least 50 units, and even more preferably at least 100 units, of enzyme per gram of polymerizable compound used in the hardenable composition. Preferably, the peroxidase enzyme is present in an amount of no greater than 2000 units per gram of polymerizable compound used in the hardenable composition.

Preferably, the oxidase substrate is present in an amount of at least 0.15 milligram (mg), more preferably at least 0.25 mg, and even more preferably at least 0.35 mg, of enzyme substrate per gram of polymerizable compound used in the hardenable composition. Preferably, the oxidase substrate is present in an amount of no greater than 10 mg per gram of polymerizable compound used in the hardenable composition.

Preferably, the reducing agent is present in an amount of at least 2 mg, more preferably at least 10 mg, and even more preferably at least 20 mg, of reducing agent per gram of polymerizable compound used in the hardenable composition. Preferably, the reducing agent is present in an amount of no greater than 50 mg per gram of polymerizable compound used in the hardenable composition.

The enzyme preparations can be prepared to contain as high as 100% pure enzyme or may contain very low levels of enzyme, for example, 1% or less. Commercial enzyme preparations usually contain 2 weight percent (wt-%) to 80 wt-% of enzyme. Thus, the compositions of the present invention will include enzymes and enzyme substrates taking into account both the activity of the enzyme preparation as well as its total amount. Generally, formulation will be based on activity, not on total weight of enzyme preparation. The level of enzyme used in the practice of this invention will depend on the enzymatic activity of the enzyme and the desired rate of hardening of the composition.

Enzymes can be used in soluble form or immobilized form. An immobilized enzyme may be used to enhance enzymatic stability and reactivity. There are many methods available for immobilization including binding on prefabricated carrier materials and incorporating into *in situ* prepared carriers. Operative binding forces vary between weak multiple adsorptive interactions and single attachments through strong covalent binding. The appropriate methods depend on the enzyme structure and application. In general, enzymes can be immobilized by attachment to carriers through either chemical reaction or physical absorption and can be used in a variety of methods as described in W. Tischer, F. Wedekind, Topics in Current Chemistry, Vol. 200, Springer, Berlin Heidelberg, 1999. Alternatively, enzymes can be encapsulated within a membrane or liposome/micelle or in solgel matrices as described in J. Am. Chem. Soc. 2002, 124, 4247-4252.

The enzymes, substrate, and reducing agent can be microencapsulated, for example, as described in U.S. Pat. No. 5,154,762 (Mitra et al.). This will generally enhance shelf stability of the hardenable composition, and if necessary permit packaging the reducing agent and enzymes together. For example, through appropriate selection of an encapsulant, the enzymes and reducing agents can be combined with the polymerizable component and optional filler and kept in a storage-stable state. Likewise, through appropriate selection of a water-insoluble encapsulant, the reducing agent and enzymes can be combined with a FAS (fluoroaluminasilicate) glass and water and maintained in a storage-stable state.

Preferably the encapsulant is a medically acceptable polymer and a good film former. Also, the glass transition temperature (Tg) of the encapsulant preferably is above room temperature.

The components of the resin system are selected such that they are miscible with the other components of the hardenable composition. That is, preferably, the components of the resin system are at least sufficiently miscible that they do not undergo substantial sedimentation when combined with the other ingredients of the composition (e.g., reducing agent and enzymes). Preferably, the components of the resin system are at least partially miscible with water. The components of the resin system can be monomers, oligomers, polymers, or combinations thereof.

The resin systems of the present invention include at least one polymerizable component. Preferably, the polymerizable component is an ethylenically unsaturated component, more preferably, a multifunctional component, and even more preferably a polymerizable ethylenically unsaturated multifunctional component

Optionally, the resin systems of the hardenable compositions of the present invention may also include an acid-functional component. The ethylenically unsaturated component can be present as a separate ingredient or the ethylenic unsaturation can, if desired, be present as a moiety in another compound such as the acid-functional component. In this way, one compound can include an acid-functional portion and an ethylenically unsaturated portion.

In one embodiment, the ethylenically unsaturated component includes α,β-unsaturated compounds. Preferred α,β-unsaturated compounds can provide altered properties such as toughness, adhesion, set time, and the like. When α,β-unsaturated compounds are employed, they preferably are water-soluble, water-miscible, or water-dispersible. Water-soluble, water-miscible, or water-dispersible (meth)acrylates (i.e., acrylates and methacrylates), (meth)acrylamides (i.e., acrylamides and methacrylamides), and urethane (meth)acrylates are preferred. Examples include, but are not limited to, 2-hydroxyethyl methacrylate, 2-hydroxypropyl methacrylate, tetrahydrofurfuryl methacrylate, glycerol mono- or di-methacrylate, trimethylol propane trimethacrylate, ethylene glycol dimethacrylate, triethylene glycol dimethacrylate, bisGMA, ethoxylated bisphenolA diacrylate, ethoxylated bisphenolA dimethacrylate, polyethylene glycol dimethacrylate, acrylamide, methacrylamide, methylene bis-acrylamide, methylene bis-methacrylamide, diacetone acrylamide, and diacetone methacrylamide. Suitable ethylenically unsaturated compounds are also available from a wide variety of commercial sources, such as Sigma-Aldrich, St. Louis, MO and Rhom and Tech, Inc., Darmstadt, Germany. Mixtures of α,β-unsaturated compounds can be used if desired.

Preferred compositions of the present invention may include a sufficient quantity of ethylenically unsaturated component to provide the desired hardening rate and desired overall properties following hardening. Preferably, the mixed but unset hardenable compositions of the invention contain at least 1 percent by weight (wt-%), more preferably at least 5 wt-%, and most preferably at least 10 wt-%, of an ethylenically unsaturated component (preferably, a multifunctional ethylenically unsaturated component), based on the total weight (including water) of the hardenable (mixed but unset) composition.

The optional acid-functional component can include monomers, oligomers, or polymers and can include oxyacid functional derivatives of carbon, phosphorous, sulfur, and boron compounds. Suitable acid-functional compounds include those listed at column 2, line 62 through column 3, line 6 of U.S. Pat. No. 4,209,434 (Wilson et al.). Preferred acid-functional compounds are polymers, including homopolymers and copolymers (i.e., of two or more different monomers), of alkenoic acids such as acrylic acid, 2-chloroacrylic acid, 2-cyanoacrylic acid, aconitic acid, citraconic acid, fumaric acid, glutaconic acid, itaconic acid, maleic acid, mesaconic acid, methacrylic acid, and tiglic acid. Mixtures of acid-functional compounds can be used if desired.

As will be appreciated by those skilled in the art, the acid-functional component should have a molecular weight sufficient to provide good storage, handling, and mixing properties. A preferred molecular weight for an acid-functional component is 60 to 100,000 weight average molecular weight as evaluated using gel permeation chromatography and a polystyrene standard, with 80 to 30,000 being most preferred

Certain embodiments of the present invention may include a sufficient quantity of an acid-functional component to provide the desired polymerization characteristics and desired overall properties following hardening. Preferably, the hardenable compositions of the invention contain at least 2 percent by weight (wt-%), more preferably at least 5 wt-%, and most preferably at least 10 wt-% of an acid-functional component, based on the total weight (including water) of the hardenable composition.

As stated above, in an alternative embodiment, the ethylenic unsaturation can be present as a moiety in the acid-functional component. For example, α,β-unsaturated acidic compounds such as glycerol phosphate monomethacrylates, glycerol phosphate dimethacrylates, hydroxyethyl methacrylate phosphates, citric acid di- or tri-methacrylates, poly(meth)acrylated oligomaleic acid, poly(meth)acrylated polymaleic acid, poly(meth)acrylated poly(meth)acrylic acid, poly(meth)acrylated polycarboxyl-polyphosphonic acid, poly(meth)acrylated polychlorophosphoric acid, poly(meth)acrylated polysulfonate, poly(meth)acrylated polyboric acid, and the like, may be used as components in the hardenable resin system. Certain of these compounds are obtained, for example, as reaction products between isocyanatoalkyl (meth)acrylates and carboxylic acids. Additional compounds of this type having both acid-functional and ethylenically unsaturated components are described in U.S. Pat. Nos. 4,872,936 (Engelbrecht) and 5,130,347 (Mitra). A wide variety of such compounds containing both the ethylenically unsaturated and acid moieties can be used. Mixtures of such compounds can be used if desired.

Other suitable free radically polymerizable compounds having ethylenically unsaturated groups include vinyl compounds such as styrene, diallyl phthalate, divinyl succinate, divinyl adipate, divinyl phthalate; siloxane-functional (meth)acrylates as disclosed, for example, in WO-00/38619 (Guggenberger et al.), WO-01/92271 (Weinmann et al.), WO-01/07444 (Guggenberger et al.), WO-00/42092 (Guggenberger et al.); and fluoropolymer-functional (meth)acrylates as disclosed, for example, in U.S. Pat. No. 5,076,844 (Fock et al.), U.S. Pat. No. 4,356,296 (Griffith et al.), EP-0373 384 (Wagenknecht et al.), EP-0201 031 (Reiners et al.), and EP-0201 778 (Reiners et al.). Mixtures of two or more free radically polymerizable compounds can be used if desired.

The hardenable compositions of the present invention can also contain fillers. Fillers may be selected from one or more of a wide variety of materials suitable for incorporation in compositions used for medical and dental applications, such as fillers currently used in dental restorative compositions, and the like. The filler is preferably finely divided. The filler can have a unimodial or polymodial (e.g., bimodal) particle size distribution. Preferably, the maximum particle size (the largest dimension of a particle, typically, the diameter) of the filler is less than 10 micrometers, and more preferably less than 2.0 micrometers. Preferably, the average particle size of the filler is less than 3.0 micrometers, and more preferably less than 0.6 micrometer.

The filler can be an inorganic material. It can also be a crosslinked organic material that is insoluble in the resin system, and is optionally filled with inorganic filler. The filler should in any event be nontoxic and suitable for use in the mouth. The filler can be radiopaque or radiolucent. The filler is also substantially insoluble in water.

Examples of suitable inorganic fillers are naturally occurring or synthetic materials including, but not limited to: quartz; nitrides (e.g., silicon nitride); glasses derived from, for example, Ce, Sb, Sn, Ba, Zn, and Al; feldspar; borosilicate glass; kaolin; talc; titania; low Mohs hardness fillers such as those described in U.S. Pat. No. 4,695,251 (Randklev); and colloidal and submicron silica particles (e.g., pyrogenic silicas such as those available under the trade designations AEROSIL, including "OX 50", "130", "150" and "200" silicas from Degussa Corp., Akron, OH and CAB-O-SIL M5 silica from Cabot Corp., Tuscola, IL). Examples of suitable organic filler particles include filled or unfilled pulverized polycarbonates, polyepoxides, and the like.

Preferred non-acid-reactive filler particles are quartz, submicron silica, and non-vitreous microparticles of the type described in U.S. Pat. No. 4,503,169 (Randklev). Mixtures of these non-acid-reactive fillers are also contemplated, as well as combination fillers made from organic and inorganic materials.

The surface of the filler particles can also be treated with a coupling agent in order to enhance the bond between the filler and the resin. The use of suitable coupling agents include gamma-methacryloxypropyltrimethoxysilane, gamma-mercaptbpropyltriethoxysilane, gamma-aminopropyltrimethoxysilane, and the like.

The filler can also be an acid-reactive filler. An acid-reactive filler is typically used in combination with an acid-functional resin component, and may or may not be used in combination with a nonreactive filler. The acid-reactive filler can, if desired, also possess the property of releasing fluoride. Suitable acid-reactive fillers include metal oxides, glasses, and metal salts. Preferred metal oxides include barium oxide, calcium oxide, magnesium oxide, and zinc oxide. Preferred glasses include borate glasses, phosphate glasses, and fluoroaluminosilicate ("FAS") glasses. FAS glasses are particularly preferred. The FAS glass preferably contains sufficient elutable cations so that a hardened dental composition will form when the glass is mixed with the components of the hardenable composition. The glass also preferably contains sufficient elutable fluoride ions so that the hardened composition will have cariostatic properties. The glass can be made from a melt containing fluoride, alumina, and other glass-forming ingredients using techniques familiar to those skilled in the FAS glassmaking art. The FAS glass preferably is in the form of particles that are sufficiently finely divided so that they can conveniently be mixed with the other cement components and will perform well when the resulting mixture is used in the mouth.

Preferably, the average particle size (typically, diameter) for the FAS glass is no greater than 10 micrometers, and more preferably no greater than 5 micrometers as measured using, for example, a sedimentation analyzer. Suitable FAS glasses will be familiar to those skilled in the art, and are available from a wide variety of commercial sources, and many are found in currently available glass ionomer cements such as those commercially available under the trade designations VTTREMER, VITREBOND, RELY.X LUTING CEMENT and KETAC-FIL (3M ESPE Dental Products, St. Paul, MN), FUJI II, GC FUJI LC and FUJI IX (G-C Dental Industrial Corp., Tokyo, Japan) and CHEMFIL Superior (Dentsply International, York, PA). Mixtures of fillers can be used if desired.

The FAS glass can optionally be subjected to a surface treatment. Suitable surface treatments include, but are not limited to, acid washing (e.g., treatment with a phosphoric acid), treatment with a phosphate, treatment with a chelating agent such as tartaric acid, and treatment with a silane or an acidic or basic silanol solution. Desirably the pH of the treating solution or the treated glass is adjusted to neutral or near-neutral, as this can increase storage stability of the hardenable composition.

In certain compositions, mixtures of acid-reactive and non-acid-reactive fillers can be used either in the same part or in different parts.

Other suitable fillers are disclosed in U.S. Pat. No. 6,387,981 (Zhang et al.) as well as International Publication Nos. WO 01/30304 (Wu et al.), WO 01/30305 (Zhang et al.), WO 01/30306 (Windisch et al.), and WO 01/30307 (Zhang et al.). Other suitable fillers are described in references cited within these publications.

The amount of filler should be sufficient to provide a hardenable composition having desirable mixing and handling properties before hardening, and good performance after hardening. Preferably, the filler represents no greater than 90 wt-%, more preferably no greater than 85 wt-%, and most preferably no greater than 80 wt-%, of the total weight (including water) of the hardenable composition. Preferably, the filler represents at least 1 wt-%, more preferably at least 5 wt-%, and most preferably at least 30 wt-%, of the total weight (including water) of the hardenable composition.

Photoinitiators can also be added to the hardenable composition, but are not required. The photoinitiator should be capable of promoting free radical crosslinking of , the polymerizable component on exposure to light of a suitable wavelength and intensity. It also preferably is sufficiently shelf-stable and free of undesirable coloration to permit its storage and use under typical dental conditions. Visible light photoinitiators are preferred. The photoinitiator preferably is miscible with the resin system, and more preferably water-soluble or water-miscible. Photoinitiators bearing polar groups usually have a sufficient degree of water-solubility or water-miscibility. The photoinitiator frequently can be used alone but typically it is used in combination with a suitable donor compound or a suitable accelerator (for example, amines, peroxides, phosphorus compounds, ketones and alpha-diketone compounds).

Suitable visible light-induced and ultraviolet light-induced initiators will be familiar to those skilled in the art. Preferred visible light-induced initiators include camphorquinone; diaryliodonium simple or metal complex salts, chromophore-substituted halomethyl-s-triazines and halomethyl oxadiazoles. Particularly preferred visible light-induced photoinitiators include combinations of an alpha-diketone such as camphorquinone, and a diaryliodonium salt such as diphenyliodonium chloride, bromide, iodide or hexafluorophosphate. Preferred ultraviolet light-induced polymerization initiators include amines that are optionally polymerizable.

If employed, the photoinitiator should be present in an amount sufficient to provide the desired rate of photopolymerization. This amount will be dependent in part on the light source, the thickness of the layer of the composition to be exposed to radiant energy and the extinction coefficient of the photoinitiator.

Preferably, mixed but unset photocurable compositions of the invention include at least 0.01 wt-%, and more preferably at least 0.1 wt-%, based on the total weight (including water) of the hardenable (mixed but unset) composition. Preferably, mixed but unset photocurable compositions of the invention include no greater than 5 wt-%, and more preferably no greater than 2 wt-%, based on the total weight (including water) of the hardenable (mixed but unset) composition.

The compositions of the invention typically contain water. Optionally, the water can be added to the initiator systems and compositions of the present invention by the end-user. The water can be distilled, deionized, or plain tap water. Generally, deionized water is preferred.

The amount of water should be sufficient to provide adequate handling and mixing properties and to permit the transport of ions, particularly in the filler-acid reaction. Preferably, water represents at least 1 wt-%, and more preferably at least 5 wt-%, of the total weight of ingredients used to form the hardenable composition. Preferably, water represents no greater than 75 wt-%, and more preferably no greater than 50 wt-%, of the total weight of ingredients used to form the hardenable composition.

Optionally, the hardenable compositions also may contain solvents (e.g., alcohols) or diluents other than water. These cosolvents are at least partially water miscible and include, for example, tetrahydrofuran, acetone, dioxane, dimethyl formamide, dimethyl sulfoxide, ethanol, methanol, propanol, isopropanol, butanol, isobutanol, ethylene glycol, ethylene glycol monomethyl ether, and propylene glycol.

The amount of cosolvent should be sufficient to provide sufficient dissolution and reactivity of the composition components. Preferably, the cosolvent represents at least 1 wt-%, and more preferably at least 5 wt-%, of the total weight of ingredients used to form the hardenable composition. Preferably, the cosolvent represents no greater than 75 wt-%, and more preferably no greater than 50 wt-%, of the total weight of ingredients used to form the hardenable composition.

If desired, the hardenable composition of the invention can contain optional additives such as pigments, inhibitors, accelerators, viscosity modifiers, surfactants, medicaments (i.e., active compounds capable of causing a desired physical or physiological change), and other ingredients that will be apparent to those skilled in the art. Exemplary desired changes that could result from an added medicament include whitening, stain bleaching, stain removing, remineralizing to form fluorapatite, plaque removal, and tartar removal. Examples of suitable medicaments include, but are not limited to, hydrogen peroxide, carbamide peroxide, sodium fluoride, sodium monophosphate, pyrophosphate, chlorhexidine, polyphosphate, triclosan, therapeutic enzymes and combinations thereof. Other useful medicaments include anti-inffammatory, antimicrobial, and other agents for treating soft tissue diseases, e.g., periodontitis treatment. The selection and amount of any one such additive can be selected by one of skill in the art to accomplish the desired result without undue experimentation.

Examples of therapeutic enzymes include those that cause (e.g., by catalysis) the decomposition of harmful carbohydrates, proteins, lipids, and/or bacterial substrates in the mouth of a subject (e.g., in the oral plaque and saliva). A preferred group of enzymes generate bactericidal products (e.g., H₂O₂). Additionally, the enzymes may contain one or more enzyme substrates to enhance therapeutic function of the enzymes.

Such enzymes are advantageously substantially active at a pH prevailing in the mouth. Typically, this is pH 5.0 to pH 9.0, more typically pH 6.0 to pH 8.5, and even more typically pH 6.4 to pH 7.5. Under certain conditions, the pH may be significantly lower, which allows for the use of a wider variety of enzymes. Oxidoreductase and hydrolase enzymes are useful classes of enzymes for use in the present invention. Oxidoreductase enzymes are those classified under the Enzyme Classification number E.C. 1 in accordance with the Recommendations (1992) of the International Union of Biochemistry and Molecular Biology (IUBMB). They catalyze oxidoreductions (i.e., redox reactions). Within the group of oxidoreductase enzymes are oxidase enzymes, peroxidase enzymes, and laccase enzymes. Oxidase enzymes catalyze the oxidation of a substrate by acting on O₂ as an acceptor of electrons and forming hydrogen peroxide. Such enzymes are classified under the enzyme classification E.C. 1.1.3, E.C. 1.2.3, E.C. 1.3.3, E.C. 1.4.3, E.C. 1.5.3. E.C. 1.7.3, E.C. 1.8.3, E.C. 1.9.3. Examples include, but are not limited to, glucose oxidase, sucrose oxidase, lactate oxidase, (S)-2-hydroxy-acid oxidase, hexose oxidase, L-or D-amino-acid oxidase, xylitol oxidase, xanthine oxidase, glycolate oxidase, L-sorbose oxidase, alcohol oxidase, gulonolactone oxidase. Corresponding enzyme substrates include, but are not limited to, β-D-glucose, sucrose, lactate, (S)-2-hydroxy-acid, broad spectrum of carbohydrates including D-glucose, D-galactose, D-mannose, maltose, lactose, and cellobiose, etc., L-or D-amino acids, xylitol, xanthine, α-hydroxy acids, L-sorbose, a primary alcohol, and L-gulono-1,4-lactone. Peroxidase enzymes act on peroxide as an acceptor of electrons. These include enzymes classified under the enzyme classification E.C. 1.11. The different types of peroxidase enzymes are distinguished by the donor molecules from which they take electrons to donate to hydrogen peroxide. In accordance with the present invention a peroxidase is used to generate free radicals from donor molecules. The donor molecules are typically capable of acting as a substrate for the peroxidase in generating such free radicals. Examples include, but are not limited to, horseradish peroxidase, soybean peroxidase, polyphenol peroxidase, manganese peroxidase, L-ascorbate peroxidase, chloroperoxidase, and iodide peroxidase. Corresponding enzyme substrates include, but are not limited to, hydrogen peroxide and electron donor molecules such as polyphenol, manganese (II), ascorbic acid, chloride, and iodide. Laccase enzymes act on O₂ and yield water without any need for peroxide. These include enzymes classified under the enzyme classification E.C. 1.10.3. Corresponding substrates include, but are not limited to, O- and P-quinols, aminophenols, and phenylenediamine. Hydrolase enzymes are those classified under the Enzyme Classification number E.C. 3 in accordance with the Recommendations (1992) of the International Union of Biochemistry and Molecular Biology (IUBMB). Within the group of hydrolase enzymes are protease enzymes, carbohydrase enzymes, and lipase enzymes. Preferred hydrolase enzymes are proteases. Protease enzymes act to break down or hydrolyze proteins. Such enzymes are classified under the classification E.C. 3.4.21, E.C. 3.4.22, E.C. 3.4.23, E.C. 3.4.24. Example include, but are not limited to, trypsin, papain, pancreatin, pepsin (e.g., pepsin A, pepsin B), chymosin, cathepsin E, gastricsin, cathepsin D, phytepsin, cyprosin, cardosin A, cardosin B, nephentesin, neurosporapepsin, saccharopepsin, renin, plasmepsin, rhodorulapepsin, acrocyclindropepsin, pycnoporopepsin, physaropepsin, aspergillopepsin, penicillopepsin, rizopuspepsin, mucorpepsin, polyproppepsin, candidaparapsin, candidapepsin, yapsin 1, yapsin 2, yapsin 3, pseudomonaspepsin, xanzhornonpepsin, thermopsin, scytalidopepsin, aleurain, omptin, lysosomale, carboxypeptidase A, cathepsin A, lysosomale pro-X carboxypeptidase, asparaginyl endopeptidase, y-glutamylhydrolase, bacillus pepstatin insensitive acid endopeptidase, carboxypeptidase, and insulysin. Of these proteases, cardosin A, candidaparapsin, pseudomonapepsin, and pepsin (particularly pepsin A) are preferred for use. Corresponding substrates for proteases are various proteins and peptides. Proteases can be used in combination with zinc to enhance anti-plaque functions. Carbohydrase enzymes act to break down or hydrolyze carbohydrates. Such enzymes are classified under the classification E.C. 3.2.1. Examples include, but are not limited to, dextranase, cellulase, amylase, α-glucosidase, β-glucosidase, lactase, invertase, amyloglucosidase, and lysozyme. Corresponding substrates for the carbohydrase enzymes include, but not limited to, dextran, cellulose, starch, oligosaccharides, beta-D-glucosides, lactose, sucrose, polysaccharides, and bacterial cell wall. Lipase enzymes act to break down or hydrolyze fatty substances, e.g., fatty acids and fatty acid esters. Such enzymes are classified under classification E.C. 3.1.1 and E.C. 3.1.4. Examples include, but are not limited to, Lipase 4000, Lipase B, Lipase 448, gastric lipase, pancreatic lipase, and plant lipase. Corresponding substrates for lipase enzymes are various fats and oils. Various combinations of enzymes and optional substrates can be used to enhance therapeutic functions. Examples include: combinations of various oxidoreductase enzymes and their corresponding substrates; combinations of glucose oxidase, glucose, and thiocyanate; and combinations of glucose oxidase/glucose dehydrogenase and glucose.

The compositions of the present invention are adjusted to provide an appropriate balance of properties in the hardenable composition, both during the polymerization reaction and after the composition has hardened. These properties include the rate of polymerization, and the shelf stability of the components of the hardenable composition. For example, the hardenable composition should preferably have a time to harden of less than or equal to 6 minutes, more preferably less than 4 minutes, and even more preferably less than 2 minutes.

The hardenable compositions of the invention can be supplied in a variety of forms including two-part powder/liquid, paste/liquid, and paste/paste systems. Other forms employing multi-part combinations (i.e., combinations of two or more parts), each of which is in the form of a powder, liquid, gel, or paste, are also possible. In a multi-part system, one part typically contains the oxidase enzyme and another part typically contains the oxidase enzyme substrate.

The components of the hardenable composition can be included in a kit, where the contents of the composition are packaged, as described below, to allow for storage of the components until they are needed.

When used as a dental composition, the components of the hardenable compositions can be mixed and clinically applied using conventional techniques. A curing light is not required (unless a photoinitiator has been included in the composition). The compositions can provide very good adhesion to dentin and/or enamel. Optionally, a primer layer can be used on the tooth tissue on which the hardenable composition is used. The compositions can also provide very good long-term fluoride release. Hence, the compositions of the invention may provide glass ionomer cements or adhesives that can be cured in bulk without the application of light or other external curing energy, do not require a pre-treatment, have improved physical properties including improved flexural strength, and have high fluoride release for cariostatic effect.

The compositions of the invention are particularly well adapted for use as a wide variety of dental materials, which may be filled or unfilled. They can be used in sealants or adhesives, which are lightly filled composites (up to 25 wt-% filler, based on the total weight of the composition) or unfilled compositions that are cured after being dispensed adjacent to a tooth (i.e., placing a dental material in temporary or permanent bonding or touching contact with a tooth). They can be used in cements, which are typically filled compositions (preferably containing greater than 25 wt-% filler and up to 60 wt-% filler). They can also be used in restoratives, which include composites that are polymerized after being disposed adjacent to a tooth, such as filling materials. They can also be used in prostheses that are shaped and polymerized for final use (e.g., as a crown, bridge, veneer, inlay, onlay, or the like), before being disposed adjacent to a tooth. Such preformed articles can be ground or otherwise formed into a custom-fitted shape by the dentist or other user.

The compositions have particular utility in clinical applications where cure of conventional light-curable cement may be difficult to achieve. Such applications include, but are not limited to, deep restorations, large crown build-ups, endodontic restorations, attachment of orthodontic brackets (including pre-coated brackets, where, for example, a paste portion could be pre-applied to the bracket and a liquid portion could later be brushed onto a tooth), bands, buccal tubes, and other devices, luting of metallic crowns or other light-impermeable prosthetic devices to teeth, and other restorative applications in inaccessible areas of the mouth.

### EXAMPLES

Objects and advantages of this invention are further illustrated by the following examples, but the particular materials and amounts thereof recited in these examples, as well as other conditions and details, should not be construed to unduly limit this invention. Unless otherwise indicated, all parts and percentages are on a weight basis, all water is deionized water, and all molecular weights are weight average molecular weight.

**Abbreviations/Definitions**

| | |
|---|---|
| AA:ITA | Copolymer made from a 4:1 mole ratio of acrylic acid:itaconic acid, prepared according to Example 3 of U.S. Pat. No. 5,130,347 (Mitra), MW (average) = 106,000; polydispersity p = 4.64. |
| IEM | 2-Isocyanatoethyl methacrylate (Sigma-Aldrich, St. Louis, MO) |
| AA:ITA:IEM | Polymer made by reacting AA:ITA copolymer with sufficient IEM to convert 16 mole percent of the acid groups of the copolymer to pendent methacrylate groups, according to the dry polymer preparation of Example 11 of U.S. Pat. No. 5,130,347. |
| HEMA | 2-Hydroxyethyl methacrylate (Sigma-Aldrich) |
| MMA | Methyl methacrylate (Sigma-Aldrich) |
| THF | Tetrahydrofuran (Sigma-Aldrich) |
| Buffer | Sodium acetate buffer (0.05 M; pH 5.2; prepared by transferring 16.67 milliters (ml) of 3 Molar (M) sodium acetate buffer solution (Sigma-Aldrich) to a 100-ml volumetric flask made to volume with deionized water). Stored at 2-8 °C |
| GOx | Glucose Oxidase Type VII-S from aspergillus niger lyophilized powder containing approximately 80% protein with an activity of 100,000-250,000 units per gram of solid (Sigma-Aldrich) |
| Peroxidase | Peroxidase Type VI-A, RZ approximately 3.0 from horseradish with an ABTS activity of approximately 1000 units per gram of solid (Sigma-Aldrich) |
| PEGDMA | Polyethyleneglycol (400) dimethacrylate; MW = 400 (Sartomer, Exton, PA) |
| GOx-S | Glucose Oxidase Solution prepared by dissolving GOx (4.2 mg) in Buffer (8 ml); equivalent to 125 units of GOx/ml solution |
| Peroxidase-S | Peroxidase Solution prepared by dissolving Peroxidase (3.8 milligrams (mg)) in Buffer (8 ml); equivalent to 625 units of Peroxidase/ml solution |
| Glucose-S | Glucose Standard Solution (100 milligrams per deciliter (mg/dl)) (Sigma-Aldrich) |
| PEGDMA-S1 | Polymer Solution prepared by dissolving PEGDMA (18 grams (g)) in water (12 ml) |
| PEGDMA-S2 | Polymer Solution prepared by dissolving PEGDMA (18.0 g) and AA:ITA:IEM (6.0 g) in water (16.0 ml) |
| PEGDMA-S3 | Polymer Solution prepared by dissolving PEGDMA (1.0 g), AA:ITA:IEM (5.0 g), and HEMA (1.0 g) in water (7.0 ml) |
| NaTS | Sodium Toluene Sulfinate (Sigma-Aldrich) |
| RA-1 | Sodium Benzene Sulfinate (Sigma-Aldrich) |
| RA-2 | Ascorbic acid (Sigma-Aldrich) |
| RA-3 * | Acetylacetone (Sigma-Aldrich) |
| RA-4 | N-phenylglycine (Sigma-Aldrich) |
| RA-5 * | Ethyl N-phenylglycinate (Sigma-Aldrich) |
| RA-6 * | p-Dimethylamino benzoic acid (Sigma-Aldrich) |
| RA-7 * | Dihydroxyethyl-p-toluidine (Sigma-Aldrich) |
| RA-8 * | 1,2,4-Trimethoxybenzene (Sigina-Aldrich) |
| RA-9 * | 1,2,4,5-Tetramethoxybenzene (Prepared by the procedure described in the Journal of American Chemical Society, Vol. 122, pp 8099-8100, 2000) |
| RA-10 | 1,3-Dimethylbarbituric acid (Sigma-Aldrich) |
| RA-11 * | Methyl-phenothiazine (Sigma-Aldrich) |
| RA-12 * | Phenothiazine (Sigma-Aldrich) |
| RA-13 * | Eosin Y (Sigma-Aldrich) |
| RA-14 * | Phenyl silane (Sigma-Aldrich) |
| RA-15 * | Dimethoxybenzylalcohol (Sigma-Aldrich) |
| RA-16 * | Tetramethyl thiourea (Sigma-Aldrich) |

| | |
|---|---|
| RA-3, RA-6 to RA9 and RA-11 to RA-16 are reference reducing agents. | |

### Polymerization Test Method

The following Polymerization Test Method (PTM) was designed to evaluate whether or not a particular reducing agent would be capable of initiating the polymerization of a polymerizable material in the presence of an oxidase, an oxidase substrate, a peroxidase, and water.

PEGDMA-S1 (1.0 g) is transferred to a 2-dram glass vial at room temperature (approximately 22°C) followed by the addition of the reducing agent (5.6 x 10⁻⁵ moles) to be evaluated. The vial is sealed with a 15-millimeter (mm) polyethylene-lined screw cap and the two components mixed. The reducing agent should be at least partially soluble in the PEGDMA-S. After mixing, the following components are sequentially added with agitation within 60 seconds: Glucose-S (0.2 ml), GOx-S (0.1 ml), and Peroxidase-S (0.1 ml). Following addition of the Peroxidase-S, the vial is capped and the components are mixed by manual shaking for 30 seconds or until gelling (i.e., polymerization and hardening) of the mixture occurs. If the mixture does not begin to gel within about 60 seconds at room temperature, then the vial is transferred to a 37°C oven and periodically monitored for gelling for up to about 60 minutes. If full or partial gelling of the mixture is observed rapidly at room temperature or within 60 minutes at 37°C, then the reducing agent evaluated is considered to be useful in an initiator system that contains an oxidase, an oxidase substrate, a peroxidase, and water.

### Example 1

A series of compositions (Samples 1 to 15) were prepared by combining at room temperature (approximately 22°C) in a glass vial the components listed in Table 1 in the following order: PEGDMA-S1, water, NaTS, Glucose-S, GOx-S, and Peroxidase-S. Following combination of the components, Sample 1 gelled instantly (i.e., polymerized to a hard solid), whereas Samples 2-15 did not gel (no visual indication of hardening for up to about 60 minutes). It is noted that only Sample 1 contained all five of the following components: glucose, glucose oxidase, peroxidase, a reducing agent (NaTS), and a polymerizable component .... (PEGDMA).

| Table 1 | | | | | | |
|---|---|---|---|---|---|---|
| PEGDMA-S1 (1 g) present in all Samples | | | | | | |
| Sample | Glucose-S (ml) | GOx-S (ml) | Peroxidase-S (ml) | NaTS (g) | Water (ml) | Gelled After Mixing |
| 1 | 0.2 | 0.1 | 0.1 | 0.01 | - | Yes |
| 2 | 0.2 | - | - | - | 0.2 | No |
| 3 | - | 0.1 | - | - | 0.3 | No |
| 4 | - | - | 0.1 | - | 0.3 | No |
| 5 | - | - | - | 0.01 | 0.4 | No |
| 6 | 0.2 | 0.1 | - | - | 0.1 | No |
| 7 | 0.2 | - | 0.1 | - | 0.1 | No |
| 8 | 0.2 | - | - | 0.01 | 0.1 | No |
| 9 | - | 0.1 | 0.1 | - | 0.2 | No |
| 10 | - | 0.1 | - | 0.01 | 0.3 | No |
| 11 | - | - | 0.1 | 0.01 | 0.3 | No |
| 12 | 0.2 | 0.1 | 0.1 | - | - | No |
| 13 | 0.2 | 0.1 | - | 0.01 | 0.1 | No |
| 14 | 0.2 | - | 0.1 | 0.01 | 0.1 | No |
| 15 | - | 0.1 | 0.1 | 0.01 | 0.2 | No |

### Example 2

A composition (Sample 16) was prepared by combining at room temperature in a glass vial the following components in the order listed: PEGDMA-S2 (1 g), NaTS (0.01 g), Glucose-S (0.2 ml), GOx-S (0.1 ml), and Peroxidase-S (0.1 ml). A second composition (Sample 17) was prepared like Sample 16, except with no enzymes (no GOx or peroxidase) and with added water (0.4 ml).

Following combination of the components, Sample 16 gelled (i.e., hardened) within a few seconds (i.e., hardened) whereas Sample 17 remained as a fluid solution with no detectable polymerization.

### Example 3

A composition (Sample 18) was prepared by combining at room temperature in a glass vial the following components in the order listed: PEGDMA-S3 (1 g), NaTS (0.01 g), Glucose-S (0.2 ml), GOx-S (0.1 ml), and Peroxidase-S (0.1 ml). A second composition (Sample 19) was prepared like Sample 18, except with no enzymes (no GOx or peroxidase) and with added water (0.4 ml).

Following combination of the components, Sample 18 gelled (i.e., hardened) in about 5 minutes whereas Sample 19 remained as a fluid solution with no detectable polymerization.

### Example 4

A series of compositions (Samples 20 to 37) were prepared by combining at room temperature (approximately 22°C) in a glass vial the following components in the order listed: PEGDMA-S1 (1 g), reducing agent or electron donor (NaTS or RA1 to RA16 in the amounts listed in Table 2), Glucose-S (0.2 ml), GOx-S (0.1 ml), and Peroxidase-S (0.1 ml). In the case of each sample, the vial was capped and the components mixed by manual shaking for 30 seconds or until curing or gelling (i.e., hardening) of the liquid mixture occurred. If samples did not begin to cure within about 60 seconds (sec) at room temperature, then they were transferred to a 37°C oven and periodically monitored for curing or gelling for up to about 30 minutes (min). The specific amounts of reducing agent or electron donor components and the evaluation of gelling results are provided in Table 2.

| Table 2 | | | | | |
|---|---|---|---|---|---|
| Sample | Reducing Agent or Electron Donor (mg) | Time to Gel (25°C) | Time to Gel (37°C) | Gel Type | Gel Color |
| 20 | None | No Gel | No Gel | - | - |
| 21 | NaTS(10) | <5 sec | - | Hard | Hazy |
| 22 | RA-1(9.2) | < 10 sec | - | Hard | Slight Haze |
| 23 | RA-2 (9.9) | < 30 sec | - | Hard | Clear |
| 24 * | RA-3 (5.6) | No Gel | No Gel | - | - |
| 25 | RA-4 (8.5) | < 60 sec | - | Hard | Clear |
| 26 * | RA-5 (10.1) | No Gel | No Gel | - | - |
| 27 * | RA-6 (9.3) | No Gel | No Gel | - | - |
| 28 * | RA-7 (11.0) | No Gel | No Gel | - | - |
| 29 * | RA-8 (9.4) | No Gel | No Gel | - | - |
| 30 * | RA-9 (11.1) | No Gel | No Gel | - | - |
| 31 | RA-10 (8.8) (0.4 ml Glucose-S) | No Gel | < 30 min | Very Hard | White |
| 32 ***** | RA-11 (12.0) | No Gel | No Gel | - | - |
| 33 * | RA-12 (11.2) | No Gel | No Gel | - | - |
| 34 * | RA-13(38.9) | No Gel | No Gel | - | - |
| 35 * | RA-14 (6.1) | No Gel | No Gel | - | - |
| 36 * | RA-15 (9.4) | No Gel | No Gel | - | - |
| 37 * | RA-16 (7.4) | No Gel | No Gel | - | - |

| | | | | | |
|---|---|---|---|---|---|
| Samples 24, 26 to 30 and 32 to 37 are reference samples | | | | | |

### Example 5

To a solution of MMA (2.86 g, 28.6 mmol) in distilled water (3.5 ml)/THF (1.5 ml) in a glass vial at room temperature were sequentially added the following components: NaTS (0.02 g), Glucose-S (0.3 ml), GOx-S (0.2 ml), and Peroxidase-S (0.2 ml). Following addition of the Peroxidase-S, the vial was capped and the components mixed by manual shaking for 30 seconds. It was observed that the mixture formed a binary solution with water-like low viscosity. The vial was then placed in a 37°C oven and periodically observed. After approximately 30 minutes, there was evidence of polymer formation in that a small clear layer appeared in the interface of the binary solution and a small amount of the clear material was observed in the lower phase. After standing at room temperature for approximately 48 hours, a separated layer of clear material was observed in the vial. It was concluded that the initiator system of NaTS, glucose, glucose oxidase, and peroxidase had polymerized the MMA monomer.

### Example 6

To a solution of HEMA (3.66 g, 28.2 mmol) in distilled water (3.5 ml)/THF (1.5 ml) in a glass vial at room temperature were sequentially added the following components: NaTS (0.02 g), Glucose-S (0.3 ml), GOx-S (0.2 ml), and Peroxidase-S (0.2 ml). Following addition of the Peroxidase-S, the vial was capped and the components mixed by manual shaking for 30 seconds to afford a homogeneous solution with water-like low viscosity. The vial was then placed in a 37°C oven and periodically observed. After approximately 5 minutes, there was evidence of polymer formation in that the viscosity of the solution began to significantly increase. After approximately 30 minutes, the viscosity of the solution was such that the liquid flowed very slowly when the vial was turned upside down. After standing at room temperature for approximately 48 hours, the solution was further thickened and phase separated with a thick clear liquid upper layer and a rubbery solid lower layer. It was concluded that the initiator system of NaTS, glucose, glucose oxidase, and peroxidase had polymerized the HEMA monomer.

## Claims

1. A free-radical initiator system comprising:
an oxidase and a peroxidase;
an oxidase substrate; and
a reducing agent that at least partially hardens a mixture of polyethyleneglycol dimethacrylate having a molecular weight of about 400, water, glucose, glucose oxidase, and horseradish peroxidase in no greater than 60 minutes at about 37°C.

2. The initiator system of claim 1 wherein the reducing agent is selected from the group consisting of sulfinic acid salts, ascorbic acid, amino acids, selected from the group consisting of cysteine, N-phenylglycine, histadine, barbituric acid derivatives, and combinations thereof.

3. The initiator system of any of claims 1 to 2 wherein the oxidase comprises glucose oxidase, lactate oxidase, hexose oxidase, glycolate oxidase, gulonolactone oxidase, L-sorbose oxidase, (S)-2-hydroxy-acid oxidase, xanthine oxidase, or combinations thereof.

4. The initiator system of any of claims 1 to 3 wherein the peroxidase comprises horseradish peroxidase, polyphenol peroxidase, manganese peroxidase, soybean peroxidase, chloroperoxidase, or combinations thereof.

5. The initiator system of any of claims 1 to 4 wherein the oxidase substrate comprises glucose, lactate, hexose, gulonolactone, L-sorbose, (S)-2-hydroxy acid, xanthine, or combinations thereof.

6. The initiator system of any of claims 1 to 5 further comprising oxygen.

7. A hardenable composition comprising:
a resin system comprising a polymerizable component; and
a free-radical initiator system according to any of claims 1 to 6.

8. The hardenable composition of claim 7 wherein the polymerizable component comprises an ethylenically unsaturated component.

9. The hardenable composition of claim 8 wherein the ethylenically unsaturated component comprises (meth)acrylates, (meth)acrylamides, or combinations thereof.

10. The hardenable composition of any of claims 7 to 9 further comprising water.

11. The hardenable composition of claim 10 further comprising a cosolvent.

12. The hardenable composition of any of claims 7 to 11 in the form of two parts.

13. The use of the hardenable composition of any of claims 7 to 12 as a dental sealant, restorative, hard and/or soft tissue coating, prosthodontic device, cement, adhesive, or periodontal treatment.

14. A method of preparing a hardened composition comprising: (a) combining a resin system comprising a polymerizable component and (b) a free-radical initiator system according to any of the claims 1 to 6 under conditions effective to harden the composition.

15. The method of claim 14 wherein the conditions effective to harden the composition comprise a temperature of 0°C to 60°C.

16. The method of claim 14 wherein the polymerizable component and the free-radical initiator system are selected such that the composition hardens at a temperature of about 25°C in less than 120 minutes.

17. The method of claim 14 wherein the hardened composition is a dental material selected from a sealant, restorative coating, prosthodontic device, cement, adhesive, or periodontal treatment.

## Patentansprüche

1. Radikalinitiatorsystem umfassend:
eine Oxidase und eine Peroxidase;
ein Oxidasesubstrat; und
ein Reduktionsmittel, das eine Mischung von Polyethylenglykoldimethacrylat mit einem Molekulargewicht von etwa 400, Wasser, Glucose, Glucoseoxidase und Meerrettichperoxidase in nicht mehr als 60 Minuten bei etwa 37 °C mindestens teilweise härtet.

2. Initiatorsystem nach Anspruch 1, wobei das Reduktionsmittel aus der Gruppe ausgewählt ist bestehend aus Sulfinsäuresalzen, Ascorbinsäure, Aminosäuren, ausgewählt aus der Gruppe bestehend aus Cystein, N-Phenylglycin, Histadin, Barbitursäurederivaten und Kombinationen derselben.

3. Initiatorsystem nach einem der Ansprüche 1 bis 2, wobei die Oxidase Glucoseoxidase, Lactatoxidase, Hexoseoxidase, Glykolatoxidase, Gulonolactonoxidase, L-Sorboseoxidase, (S)-2-Hydroxysäureoxidase, Xanthinoxidase oder Kombinationen derselben umfasst.

4. Initiatorsystem nach einem der Ansprüche 1 bis 3, wobei die Peroxidase Meerrettichperoxidase, Polyphenolperoxidase, Manganperoxidase, Sojabohnenperoxidase, Chlorperoxidase oder Kombinationen derselben umfasst.

5. Initiatorsystem nach einem der Ansprüche 1 bis 4, wobei das Oxidasesubstrat Glucose, Lactat, Hexose, Gulonolacton, L-Sorbose, (S)-2-Hydroxysäure, Xanthin oder Kombinationen derselben umfasst.

6. Initiatorsystem nach einem der Ansprüche 1 bis 5, welches des Weiteren Sauerstoff umfasst.

7. Härtbare Zusammensetzung umfassend:
ein Harzsystem umfassend eine polymerisierbare Komponente; und
ein Radikalinitiatorsystem nach einem der Ansprüche 1 bis 6.

8. Härtbare Zusammensetzung nach Anspruch 7, wobei die polymerisierbare Komponente eine ethylenisch ungesättigte Komponente umfasst.

9. Härtbare Zusammensetzung nach Anspruch 8, wobei die ethylenisch ungesättigte Komponente (Meth)acrylate, (Meth)acrylamide oder Kombinationen derselben umfasst.

10. Härtbare Zusammensetzung nach einem der Ansprüche 7 bis 9, welche des Weiteren Wasser umfasst.

11. Härtbare Zusammensetzung nach Anspruch 10, welche des Weiteren ein Co-Lösungsmittel umfasst.

12. Härtbare Zusammensetzung nach einem der Ansprüche 7 bis 11, in Form von zwei Teilen.

13. Verwendung der härtbaren Zusammensetzung nach einem der Ansprüche 7 bis 12 als Dentaldichtungsmittel, Instandsetzungsmittel, Beschichtung für Weich- und/oder Hartgewebe, zahnärztliche Prothetik, Zement, Klebstoff oder periodontale Behandlung.

14. Verfahren zum Herstellen einer gehärteten Zusammensetzung umfassend: (a) das Kombinieren eines Harzsystems umfassend eine polymerisierbare Komponente und (b) eines Radikalinitiatorsystems nach einem der Ansprüche 1 bis 6 unter Bedingungen, die zum Härten der Zusammensetzung wirksam sind.

15. Verfahren nach Anspruch 14, wobei die Bedingungen, die zum Härten der Zusammensetzung wirksam sind, eine Temperatur von 0 °C bis 60 °C umfassen.

16. Verfahren nach Anspruch 14, wobei die polymerisierbare Komponente und das Radikalinitiatorsystem so ausgewählt werden, dass die Zusammensetzung bei einer Temperatur von etwa 25 °C in weniger als 120 Minuten härtet.

17. Verfahren nach Anspruch 14, wobei die gehärtete Zusammensetzung ein Dentalmaterial ist ausgewählt aus einem Dichtungsmittel, einer instandsetzenden Beschichtung, zahnärztlicher Prothetik, Zement, Klebstoff oder einer periodontalen Behandlung.

## Revendications

1. Système initiateur à radicaux libres, comprenant :
une oxydase et une peroxydase ;
un substrat d'oxydase ; et
un agent réducteur qui durcit au moins en partie un mélange de diméthacrylate de polyéthylène glycol ayant une masse moléculaire d'environ 400, d'eau, de glucose, de glucose-oxydase, et de peroxydase du raifort, dans un délai ne dépassant pas 60 minutes à environ 37 °C.

2. Système initiateur selon la revendication 1, dans lequel l'agent réducteur est sélectionné dans le groupe constitué des sels d'acide sulfinique, de l'acide ascorbique, des acides aminés, sélectionnés dans le groupe constitué de la cystéine, de la N-phénylglycine, de l'histadine, des dérivés d'acide barbiturique, et de combinaisons de ceux-ci.

3. Système initiateur selon l'une quelconque des revendications 1 à 2, dans lequel l'oxydase comprend la glucose-oxydase, la lactate-oxydase, l'hexose-oxydase, la glycolate-oxydase, la gulonolactone-oxydase, la L-sorbose-oxydase, la (S)-2-hydroxy-acide oxydase, la xanthine-oxydase, ou des combinaisons de celles-ci.

4. Système initiateur selon l'une quelconque des revendications 1 à 3, dans lequel la peroxydase comprend la peroxydase du raifort, la polyphénol-peroxydase, la manganèse-peroxydase, la peroxydase du soja, la chloroperoxydase, ou des combinaisons de celles-ci.

5. Système initiateur selon l'une quelconque des revendications 1 à 4, dans lequel le substrat d'oxydase comprend le glucose, le lactate, l'hexose, le gulonolactone, le L-sorbose, le (S)-2-hydroxy-acide, la xanthine, ou des combinaisons de ceux-ci.

6. Système initiateur selon l'une quelconque des revendications 1 à 5, comprenant en outre l'oxygène.

7. Composition durcissable, comprenant :
un système de résine comprenant un composant polymérisable ; et
un système initiateur à radicaux libres selon l'une quelconque des revendications 1 à 6.

8. Composition durcissable selon la revendication 7, dans laquelle le composant polymérisable comprend un composant à insaturation éthylénique.

9. Composition durcissable selon la revendication 8, dans laquelle le composant à insaturation éthylénique comprend des (méth)acrylates, des (méth)acrylamides, ou des combinaisons de ceux-ci.

10. Composition durcissable selon l'une quelconque des revendications 7 à 9, comprenant en outre de l'eau.

11. Composition durcissable selon la revendication 10, comprenant en outre un co-solvant.

12. Composition durcissable selon l'une quelconque des revendications 7 à 11, sous forme de deux parties.

13. Utilisation de la composition durcissable selon l'une quelconque des revendications 7 à 12, comme matériau d'étanchéité dentaire, matériau de restauration, revêtement de tissus durs et/ou mous, dispositif prosthodontique, ciment, adhésif, ou traitement périodontal.

14. Méthode de préparation d'une composition durcie, comprenant : (a) la combinaison d'un système de résine comprenant un composant polymérisable et (b) un système initiateur à radicaux libres selon l'une quelconque des revendications 1 à 6 dans des conditions efficaces pour durcir la composition.

15. Méthode selon la revendication 14, dans laquelle les conditions efficaces pour durcir la composition comprennent une température de 0 °C à 60 °C.

16. Méthode selon la revendication 14, dans laquelle le composant polymérisable et le système initiateur à radicaux libres sont sélectionnés de sorte que la composition durcisse à une' température d'environ 25 °C en moins de 120 minutes.

17. Méthode selon la revendication 14, dans laquelle la composition durcie est un matériau dentaire sélectionné parmi un matériau d'étanchéité, un revêtement de restauration, un dispositif prosthodontique, un ciment, un adhésif, ou un traitement périodontal.
